# EUROPEAN PATENT SPECIFICATION

(11) **EP 4 534 066 B1**
(45) Date of publication and mention of the grant of the patent: **04.02.2026**
(21) Application number: 23201865.5
(22) Date of filing: 05.10.2023
(51) Int. Cl.: A61J 15/00, A61B 34/20

(54) **NASOGASTRIC DEVICE AND SYSTEM THEREOF**
NASENMAGENVORRICHTUNG UND SYSTEM DAFÜR
DISPOSITIF NASOGASTRIQUE ET SYSTÈME ASSOCIÉ

(43) Date of publication of application: 09.04.2025
(73) Proprietor: Perren, Andreas, 6500 Bellinzona (CH)
(72) Inventor: Perren, Andreas, 6500 Bellinzona (CH); Poretti, Samuel, Chiasso (CH); Hajdaj, Fatlind, Lugano (CH)
(74) Representative: Praxi Intellectual Property Milano

(56) References cited:
- WO-A1-2023/119296
- US-A- 6 072 383
- US-A1- 2003 004 411
- US-A1- 2007 265 690
- US-A1- 2023 285 249
- US-A1- 2023 297 802

## Description

### TECHNICAL FIELD OF THE INVENTION

The present invention relates to a nasogastric device for enteral feeding and relative localization system. The nasogastric tube of the present invention is provided with a passive resonant tag, and the system further comprises a reader device configured to stimulate the resonant tag and detect the response of the latter. It forms subject matter of the present invention also a method for localizing the nasogastric device comprising: inducing the resonance of the tag by means of a magnetic field and detecting the presence and the position of the tube based on the variation of said magnetic field caused by the tag.

### STATE OF THE ART

Doctors and nurses are faced every day with patients who need artificial nutrition. For this purpose, in the common clinical practice, a nasogastric tube is used to provide nutrition through the esophagus canal until the stomach.

When this type of intervention is performed, it is crucial to be able to know the position of the probe inside the stomach. To this aim, currently, an abdominal X-ray is performed to identify the position of the nasogastric probe within the body. Such type of methodology is potentially harmful other than to be expensive, time consuming and, potentially, causing delay in the administration of drugs.

For these reasons, recently, alternative X-ray free methods for gastro-intestinal tube placement have been proposed, namely the Electromagnetic Placement Device (EPD) using electromagnetic sensors. In this context, WO2007002084A discloses a position-sensing medical tube coupled with a sensor electronics. The position-sensing medical tube comprises a medical tube and position-sensor apparatus. The position-sensor apparatus is adapted to communicate with the sensor electronics so as to provide information dependent on the relative position of the medical tube. The movement of the medical tube, and thus, of the coupled position-sensor apparatus is sensed by the sensor electronics. The sensor electronics interprets state-data as that the medical tube has moved and responds in a predetermined way, such as triggering an alarm and turning off a process. CN109864892A discloses an enteral feeding tube provided with a sensor cavity comprising an electromagnetic sensor formed by a sensor body including a core positioned at a distal end of the sensor cavity, and a wire extending along a length of the sensor cavity. US2022117676A1 discloses a system for positioning a device for enteral feeding comprising: an electromagnetic field generator configured to generate an electromagnetic field covering a treatment area, an enteral feeding tube comprising an electromagnetic sensor, the electromagnetic sensor configured to receive signals indicative of the electromagnetic field. The system comprises also a processing circuitry configured to load an X-ray, CT, ultrasound or MRI image of the subject's chest, co-register on such images, a first and a second anatomic landmarks marked on the subject's torso, and to display, on the images, a path of the insertion device insertion, the path being generated according to changes in the strength of the electromagnetic field sensed by the tip sensor's during the insertion of the tube.US20230285249A1 describes a nasogastric device comprising a feeding tube and a marker positioned on such a tube. The tube includes: a feeding lumen for supplying substances or pressure to a subject's stomach and/or duodenum, through the esophagus; and a sensor lumen, the sensor lumen comprising an electromagnetic sensor. The latter includes: a sensor body comprising a core positioned at the distal end of the tube; and a wire extending along the length of the sensor lumen. Disadvantageously, in addition to the complexity of the system due to the presence of a double lumen, the sensor body requires an external electrical connection towards a measuring and visualizing unit and the system also comprises reference sensors configured to be placed on the patient's thorax. US20070265690 discloses a system for catheter position tracking, comprising: one or more field generators, which are arranged to generate one or more respective position-varying magnetic fields; one or more transponder contained in the catheter, the transponder comprising a resonant circuit having a resonance frequency and comprising a field-responsive element, which is operative to vary the resonance frequency responsively to the one or more magnetic fields; and a position tracker, which is arranged to remotely sense the resonance frequency of the resonant circuit and to determine a position of the transponder responsively to the sensed resonance frequency. Disadvantageously, the field generators are location pads that are to be placed on the patient's body.

The costs associated with the aforementioned techniques are enormous and often, as in US2022117676A1, exposure to ionizing radiation is still required. Moreover, the cited priori art EPDs employ an active electromagnetic component placed on the insertion tube powered by a battery or by an external source of energy.

### OBJECTS AND SUMMARY OF THE INVENTION

The invention is disclosed in the claims.

A first object of the present invention is, thus, to provide a nasogastric device for enteral feeding that is able to be localized without using radiation potentially harmful such as X-rays.

A second object of the present invention is to provide a nasogastric localization system that is able to localize in real time the nasogastric tube so to allow real-time correction of the tube position during of the insertion procedure.

A third object of the present invention is to provide a nasogastric device for enteral feeding and relative localization system that is less complex and less expensive than the prior art systems.

To this aims the nasogastric device of the present invention comprises:
- a nasogastric tube; and
- a tag placed on the flexible tube.

The tag is a passive resonant structure having a resonance frequency band and is configured to interfere with a time varying magnetic field external to the device by changing its amplitude and/or its spatial distribution, the external magnetic field having a frequency comprised in the resonance frequency band.

A passive resonant tag permits a rapid check of the correct tube position in order to avert displacement or usage outside the stomach and can be also clearly distinguished from other metallic objects, namely surgical metal clips, other metallic medical devices or electric wires for pacemaker/ defibrillator or implants.

The aforementioned nasogastric device is part of system for enteral feeding comprising, other than nasogastric device, also a reader device that, in its turn, comprises: means for generating a source time varying magnetic field having a frequency comprised in the resonance frequency band; and means for detecting a derived time varying magnetic field. The tag of the nasogastric device will interfere with the source magnetic field, by changing its amplitude and/or spatial distribution in such a way to originate the derived magnetic field having its own amplitude and distribution. The basic components of the tag and the reader device can be one or more inductors and, eventually one or more capacitors. The simplicity of the proposed electronic equipment allows the development of an effective product at low cost. The present invention provides also for a method for localizing a nasogastric tube comprising:
- generating a source time varying magnetic field external to the nasogastric tube having its own amplitude and its own spatial distribution; and
- detecting a derived magnetic field resulting from an interaction of the first source magnetic field with a surrounding environment;

The interaction of the source magnetic field with the surrounding environment comprises:
- inducing the resonance of a passive resonance structure placed on the tube and having a resonance frequency band, by means of the source magnetic field, said source magnetic field having a frequency comprised in the resonance frequency band; and
- changing, the amplitude and/or the distribution of the source magnetic field, by means of the passive resonance structure, originating the derived magnetic field.

The method of the present invention can also comprise the step of moving the means for detecting the derived magnetic field in proximity to the chest, abdomen or back of the patient in such a way to localize the nasogastric tube when the signal generated by the means for derived magnetic field is above a given threshold.

Finally, a further object of the present invention is also to provide a system and a method that is able to localize a nasogastric tube also without moving parts of the systems.

To this aim, the means for generating the source magnetic field can comprise also:
- a bidimensional array of inductors placed at different distance from the tag, each of the inductor of the array being placed at its own distance from the tag and producing an output signal dependent on the amplitude and on the distribution of the derived time varying magnetic field; and
- a processor configured for:
   - calculating a maximum value of the output signals;
   - identifying an inductor of interest as the inductor of the array producing the maximum value; and
   - localizing the tag based on the distance of the inductor of interest from the tag.

Through the bidimensional array of inductors, it is possible to locate the tag by analysing the amplitude of the output signals produced by each inductor. By comparing them, the inductor showing the maximum response is individuated as the narrowest inductor to the tag. The tag position is, thus, calculated based on the known distance of the narrowest inductor from the tag.

These and further features of the present invention will be made clearer by the reading the following detailed description, relating to some preferred embodiments of the present invention, to be considered by way of a non-limiting example of the more general concepts claimed.

### BRIEF DESCRIPTION OF THE DRAWINGS

The following description refers to the accompanying drawings, in which:
- Figure 1 is a representation of the system according to the present invention;
- Figure 2 is a schematic representation of the transmitter inductor and the receiver inductor of the reader device of the system according to the present invention;
- Figure 3a is a side view of a second embodiment of the device of the present invention;
- Figure 3b is a top view of a second embodiment of the device of the present invention;
- Figure 3c is a bottom view of a second embodiment of the device of the present invention;
- Figure 4a is a side view of a third embodiment of the device of the present invention;
- Figure 4b is a top view of a third embodiment of the device of the present invention;
- Figure 4c is a bottom view of a third t embodiment of the device of the present invention;
- Figure 5 is a side view of a fourth embodiment of the device of the present invention;
- Figure 6 is a diagram comprising a first curve representing the output signal of the reader device induced by the resonance of the tag versus the frequency (continuous line) and a second curve representing the output signal of the reader device in absence of the tag versus the frequency (dashed line);
- Figure 7a is a schematic representation of the induction balance simulation model used in the preliminary simulations of the system according to the present invention;
- Figure 7b is curve representing the direct coupling voltage of the receiver inductor in function of the distance between the centres of the transmitter and the receiver inductors in the preliminary simulations of the system according to the present invention;
- Figure 8a is schematic representation of the induction balance simulation model used in the "small tag" simulations of the system according to the present invention;
- Figure 8b is a curve representing the voltage induction on the receiver inductor vs the tag vertical distance from the receiver inductor, in the "small tag" simulations of the system according to the present invention;
- Figure 9 is a circuit model for the tag in the "small tag" simulations of the system according to the present invention;
- Figure 10a is a curve representing the voltage induction on the receiver inductors vs the tag vertical distance from the receiver inductor, in the example wherein both the transmitter inductor and the tag inductors are resonating, in the "small tag" simulations of the system according to the present invention;
- Figure 10b shows the voltage induction on the receiver inductors vs frequency, in the example wherein both the transmitter inductor and the tag inductor are resonating, in the "small tag" simulations of the system according to the present invention. The continuous curve is referred to a vertical distance between the tag and the receiver inductor of 10 mm, the dotted curve is referred to a vertical distance between the tag and the receiver inductor of 50 mm and the dashed curve is referred to a vertical distance between the tag and the receiver inductor of 140 mm;
- Figure 11a is a curve representing the voltage induction on the receiver inductors vs the tag vertical distance from the receiver inductor, in the example wherein the transmitter inductor, the receiver inductor and the tag inductors are resonating, in the "small tag" simulations of the system according to the present invention;
- Figure 11b shows the voltage induction on the receiver inductors vs frequency, in the example wherein the transmitter inductor, the receiver inductor and the tag inductor are resonating, in the "small tag" simulations of the system according to the present invention. The continuous curve is referred to a vertical distance between the tag and the receiver inductor of 10 mm, the dotted curve is referred to a vertical distance between the tag and the receiver inductor of 50 mm and the dashed curve is referred to a vertical distance between the tag and the receiver inductor of 140 mm;
- Figure 12a is schematic representation of the induction balance simulation model used in the "iron tag" simulations of the system according to the present invention;
- Figure 12b is a curve representing the voltage induction on the receiver inductor vs the iron sheet vertical distance from the receiver inductor, in the "iron tag" simulations of the system according to the present invention;
- Figure 13a shows the voltage induction on the receiver inductors vs frequency, in the example wherein both transmitter inductor and the receiver inductor are resonating, in the "iron tag" simulations of the system according to the present invention. The continuous curve is referred to a vertical distance between the iron sheet and the receiver inductor of 10 mm, the dotted curve is referred to a vertical distance between the iron sheet and the receiver inductor of 50 mm and the dashed curve is referred to a vertical distance between the iron sheet and the receiver inductor of 140 mm;
- Figure 13b is a diagram relative to the "iron tag" simulations, comprising a first curve representing the output signal of the reader device induced by the resonance of the tag versus the frequency (continuous line), a second curve representing the output signal of the reader device induced by the resonance of the iron sheet versus the frequency (dashed line) and a third curve representing the output signal of the reader device in absence of the tag versus the frequency (dotted line);
- Figure 14 is schematic representation of the induction balance simulation model used in simulations for evaluating the reader device locating accuracy;
- Figure 15a is a curve representing shows the voltage induction on the receiver inductors vs frequency with a tag sweep along x axis; and
- Figure 15b is a curve representing shows the voltage induction on the receiver inductors vs frequency with a tag sweep along y axis.

### DETAILED DESCRIPTION OF THE INVENTION

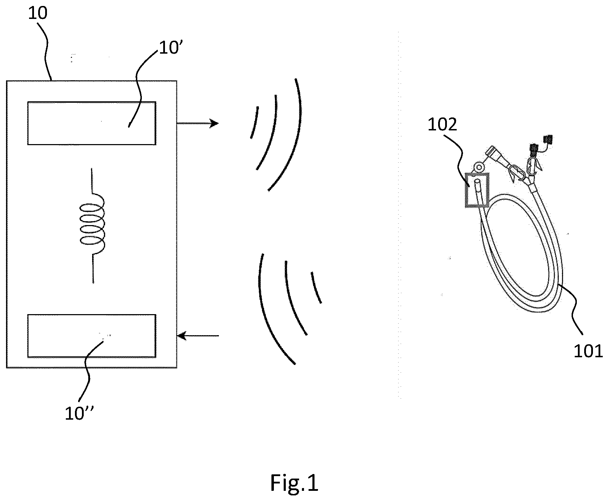

Referring to Figure 1, a first embodiment of the device of the present invention comprises:
- a nasogastric tube (101); and
- a tag (102) placed on the flexible tube (101).

The tag (102) is a passive resonant structure having a resonance frequency band, and is configured to interfere with a time varying magnetic field external to the device by changing its amplitude and/or its spatial distribution, the external magnetic field having a frequency comprised in the resonance frequency band.

The tag (102) is placed on the distal end of the tube (101) and comprises one inductor constituted by a coil wrapped around the tube and having a resonance frequency band with a central frequency comprised between 100 kHz and 5 MHz, the central frequency being, preferably, equal to 1 MHz.

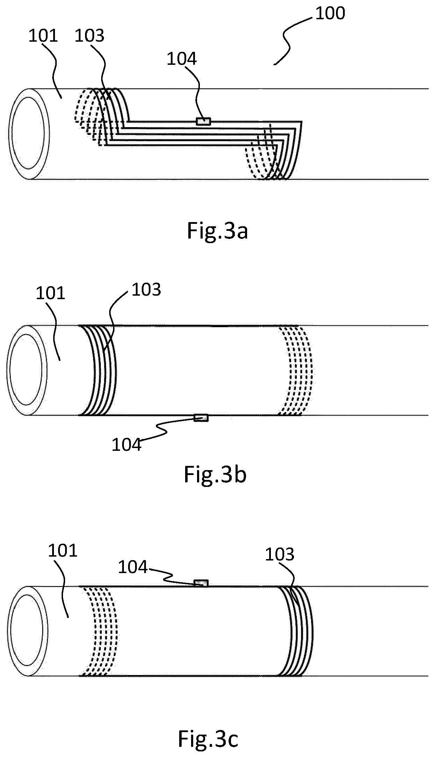

Referring to Figure 1, 3a, 3b, 3c, a second embodiment of the device (100) of the present invention comprises:
- a nasogastric tube (101); and
- a tag (102) placed on the flexible tube (101).

The tag (102) is a passive resonant structure having a resonance frequency band, and is configured to interfere with a time varying magnetic field external to the device (100) by changing its amplitude and/or its spatial distribution, the external magnetic field having a frequency comprised in the resonance frequency band.

The tag (102) is placed on the distal end of the tube (101) and comprises one capacitor (104) and one inductor (103) constituted by a coil (103), having a resonance frequency band with a central frequency comprised between 100 kHz and 5 MHz, the central frequency being, preferably, equal to 1 MHz.

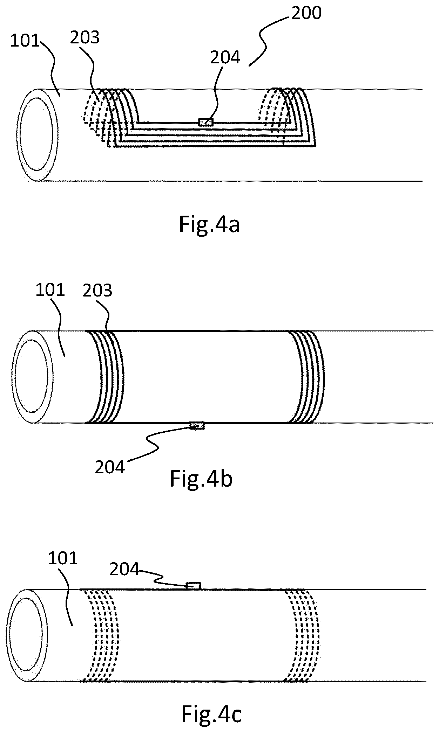

The coil (103) is wrapped around the tube (101) and, as shown in figures 3a, 3b and 3c, comprises a first curvilinear portion of wires, a second curvilinear portion of wires and rectilinear portion of wires, that is parallel to the central axis of the tube and is comprised between the curvilinear portions. The two curvilinear portions of wires are wrapped around the tube (101) in such a way to be symmetrical with respect to central axis of the tube (101). The capacitor (104) is placed in the middle point of the rectilinear portion of wires. Referring to Figure 1, 4a, 4b, 4c, a third embodiment of the device (200) of the present invention comprises:
- a nasogastric tube (101); and
- a tag (102) placed on the flexible tube (101).

The tag (102) is a passive resonant structure having a resonance frequency band and is configured to interfere with a time varying magnetic field external to the device (200) by changing its amplitude and/or the spatial distribution, the external magnetic field having a frequency comprised in the resonance frequency band.

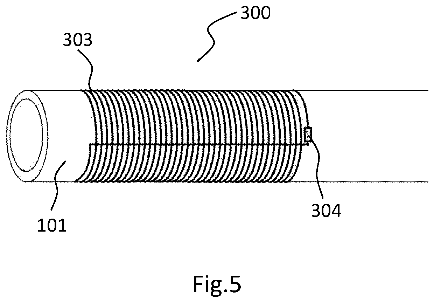

The tag (102) is placed on the distal end of the tube (101) and comprises one capacitor (204) and one inductor (203) constituted by a coil (203), having a resonance frequency band with a central frequency comprised between 100 kHz and 5 MHz, the central frequency being, preferably, equal to 1 MHz. The coil (203) is wrapped around the tube (201) and, as shown in figures 4a, 4b and 4c, comprises a first curvilinear portion of wires, a second curvilinear portion of wires and a rectilinear portion of wires, that is parallel to the central axis of the tube and is comprised between the curvilinear portions. The two curvilinear portions of wires are wrapped around the tube (101) in such a way to be symmetrical with respect to an axis perpendicular to the central axis of the tube (101). The capacitor (204) is placed in the middle point of the rectilinear portion of wires. Referring to Figure 1, and 5 a fourth embodiment (300) of the device of the present invention comprises:
- a nasogastric tube (101); and
- a tag (102) placed on the flexible tube (101).

The tag (102) is a passive resonant structure having a resonance frequency band, and is configured to interfere with a time varying magnetic field external to the device (300) by changing its amplitude and/or its spatial distribution, the external magnetic field having a frequency comprised in the resonance frequency band.

The tag (102) is placed on the distal end of the tube (101) and comprises one capacitor (304) and one inductor (303) constituted by a coil (303), having a resonance frequency band with a central frequency comprised between 100 kHz and 5 MHz, the central frequency being, preferably, equal to 1 MHz. The coil (103), as shown in figure 5, is wrapped around the tube (101) in a circumferential manner so to cover almost all the distal end of the tube (101).

In all the four embodiments mentioned above, the capacitor (104, 204, 304) is, preferably, a surface mount technology capacitor. In alternative the capacitor (104, 204, 304) can be also an interdigital capacitor. The preferred material used for the tag (101) is biocompatible and non-ferromagnetic material, in order to guarantee full compatibility with diagnostic systems based on Magnetic Resonance Imaging (MRI). Among different possible conductive materials with such characteristics, such as titanium-vanadium alloys, copper, low carbon stainless steel, titanium and silver paste, copper is the preferred material for the resonant tag since it shows the highest quality factor among the others, having the narrower and sharper resonance peak in the frequency response.

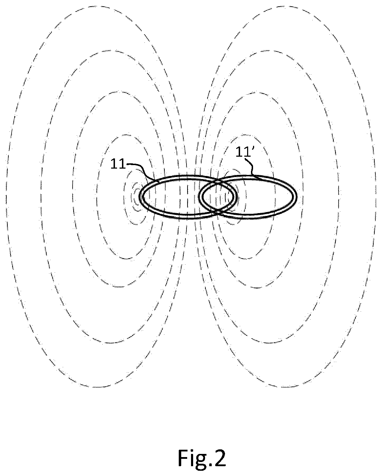

Referring to Figure 1 and 2, a first embodiment of the system of the present invention comprises:
- a nasogastric tube (101):
- a tag (102) placed on the flexible tube (101); and
- a reader device (10) comprising:
   - means (10') for generating a source time varying magnetic field; and
   - means (10") for detecting a derived time varying magnetic field;

The tag (102) can have the characteristics of the tag (102) already described above, relatively to the first, second, third and fourth embodiment of the device (100, 200, 300) according to the present invention.

The means (10') for generating the source magnetic field comprise an inductor (11), or transmitter inductor (11) and the means (10") for detecting the derived magnetic field comprise another inductor (11'), or receiver inductor (11'). As shown in Figure 2, the transmitter inductor (11) and the receiver inductor (11') partially overlap. The transmitter inductor (11) produces a magnetic field that induces a voltage, by Faraday Law, on the receiver inductor (11'). Thanks to the fact that the first inductor (11) and the second inductor (11') overlap only partially, the induced voltage on the second inductor (11') can be canceled out. This "equilibrium" situation can be easily overturned by introducing some external object such as the tag (102). In such a case, due to the slightly modified magnetic field distribution, the receiver inductor (11') comes out of its "equilibrium" and its induced voltage is now no longer zero.

Referring to Figure 1, a second embodiment of the system of the present invention comprises:
- a nasogastric tube (101):
- a tag (102) placed on the flexible tube (101); and
- a reader device (10) comprising:
   - means (10') for generating a source time varying magnetic field; and
   - means (10") for detecting a derived time varying magnetic field;

The tag (102) can have the characteristics of the tag (102) already described above, relatively to the first, second, third and fourth embodiment of the device (100, 200, 300) according to the present invention.

The means (10') for generating the source magnetic field comprise an inductor (11), or transmitter inductor (11) and a capacitor and the means (10") for detecting the derived magnetic field comprise another inductor (11'), or receiver inductor (11').

Referring to Figure 1, a third embodiment of the system of the present invention comprises:
- a nasogastric tube (101):
- a tag (102) placed on the flexible tube (101); and
- a reader device (10) comprising:
   - means (10') for generating a source time varying magnetic field; and
   - means (10") for detecting a derived time varying magnetic field;

The tag (102) can have the characteristics of the tag (102) already described above, relatively to the first, second, third and fourth embodiment of the device (100, 200, 300) according to the present invention.

The means (10') for generating the source magnetic field comprise an inductor (11), or transmitter inductor (11), while the means (10") for detecting the derived magnetic field comprise another inductor (11'), or receiver inductor (11') and a capacitor. Referring to Figure 1, a fourth embodiment of the system of the present invention comprises:
- a nasogastric tube (101):
- a tag (102) placed on the flexible tube (101); and
- a reader device (10) comprising:
   - means (10') for generating a source time varying magnetic field; and
   - means (10") for detecting a derived time varying magnetic field;

The tag (102) can have the characteristics of the tag (102) already described above, relatively to the first, second, third and fourth embodiment of the device (100, 200, 300) according to the present invention.

The means (10') for generating the source magnetic field comprise an inductor (11), or transmitter inductor (11) and a first capacitor. Even the means (10") for detecting the derived magnetic field comprise another inductor (11'), or receiver inductor (11') and a second capacitor.

In all the embodiments of the systems described above (from the first to the fourth), and as detailed in the following paragraphs relative to the methods according to the present invention, when the system is in use, the reader (10) can be moved in proximity to the chest and abdomen of the patient and, when the response of the reader is above a given threshold, the nasogastric tube (101) is considered as localized.

Referring to Figure 1, a fifth embodiment of the system of the present invention comprises:
- a nasogastric tube (101):
- a tag (102) placed on the flexible tube (101); and
- a reader device (10) comprising:
   - means (10') for generating a source time varying magnetic field; and
   - means (10") for detecting a derived time varying magnetic field;

The tag (102) can have the characteristics of the tag (102) already described above, relatively to the first, second, third and fourth embodiment of the device (100, 200, 300) according to the present invention.

The means (10') for generating the source magnetic field comprise an inductor (11), or transmitter inductor (11). Together to the transmitter inductor, the means (10') for generating the source magnetic field can comprise also a capacitor. The means (10") for detecting the derived magnetic field, instead, comprise:
- a bidimensional array of inductors placed at different distance from the tag (102), each of the inductor of the array being placed at its own distance from the tag (102) and producing an output signal dependent on the amplitude and on the distribution of the derived time varying magnetic field; and
- a processor configured for:
   - calculating a maximum value of the output signals;
   - identifying an inductor of interest as the inductor of the array producing the maximum value; and
   - localizing the tag (102) based on the distance of the inductor of interest from the tag (102).

As previously mentioned, thanks to the presence of the bidimensional array of inductors, it is possible to locate the tag without moving the reader (10).

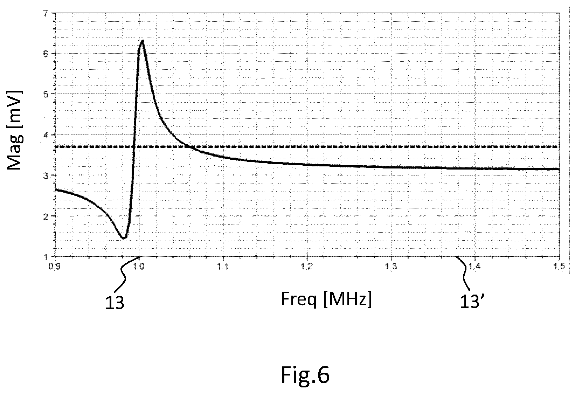

Referring to Figures 1 and 6, a first embodiment of the method according to the present invention, comprises:
- generating a first source time varying magnetic field external to the nasogastric tube (101), the first source magnetic field having a first frequency (13) comprised in the resonance frequency band (102);
- detecting a first derived magnetic field resulting from an interaction of the first source magnetic field with the surrounding environment, the first derived magnetic field having an amplitude;
- generating a second source time varying magnetic field external to the nasogastric tube (101), the second source magnetic field having a second frequency (13') outside from the resonance frequency band (102);
- detecting a second derived magnetic field resulting from an interaction of the first source magnetic field with the surrounding environment, the second derived magnetic field having an amplitude;
- determining, by means of a processor, the presence of the passive resonance structure (102) based on the difference between the amplitude of the first derived magnetic field and the amplitude of the second derived magnetic field, said determining comprising:
   - calculating a difference between the amplitude of the first derived magnetic field and the amplitude of the second derived magnetic field;
   - comparing the difference with a fixed threshold;
   - identifying the passive resonance structure as present, if the difference is above the fixed threshold; and
   - identifying the passive resonance structure as absent, if the difference is below the fixed threshold.

Obviously, it can used also a number of frequencies higher than two to generate the source magnetic field and it is possible to compare a number of derived magnetic fields higher than two. For example the method of the present invention can comprise the following steps:
- generating a first source time varying magnetic field external to the nasogastric tube (101), the first source magnetic field having a first frequency (13) comprised in the resonance frequency band (102);
- detecting a first derived magnetic field resulting from an interaction of the first source magnetic field with the surrounding environment, the first derived magnetic field having an amplitude;
- generating a second source time varying magnetic field external to the nasogastric tube (101), the second source magnetic field having a second frequency (13') outside from the resonance frequency band (102);
- detecting a second derived magnetic field resulting from an interaction of the first source magnetic field with the surrounding environment, the second derived magnetic field having an amplitude;
- generating a third source time varying magnetic field external to the nasogastric tube (101), the third source magnetic field having a third frequency outside from the resonance frequency band (102);
- detecting a third derived magnetic field resulting from an interaction of the first source magnetic field with the surrounding environment, the second derived magnetic field having an amplitude; and
- determining, by means of a processor, the presence of the passive resonance structure (102) based on the difference between the amplitude of the first derived magnetic field and the amplitude of the second derived magnetic field and the difference between the amplitude of the first derived magnetic field and the amplitude of the third derived magnetic field, said determining comprising:
   - calculating a first difference between the amplitude of the first derived magnetic field and the amplitude of the second derived magnetic field;
   - calculating a second difference between the amplitude of the first derived magnetic field and the amplitude of the third derived magnetic field;
   - comparing the first and the second difference with a fixed threshold; and
   - identifying the passive resonance structure as present, if both the first and the second difference is above the fixed threshold; or if at least one among the first and the second difference is above the threshold.

Referring to Figure 1, a second embodiment of the method according to the present invention, comprises:
- generating a first source time varying magnetic field external to the nasogastric tube (101), the first source magnetic field having a frequency comprised in the resonance frequency band;
- detecting, by using means (10") for detecting a derived time varying magnetic field, a first derived magnetic field resulting from an interaction of the first source magnetic field with the surrounding environment, the means (10") for detecting a derived time varying magnetic field being in a first position and the first derived magnetic field having an amplitude;
- moving the means (10") for detecting a derived time varying magnetic field, from a first position to a second position;
- generating a second source time varying magnetic field external to the nasogastric tube (101), the second source magnetic field having a frequency comprised in the resonance frequency band;
- detecting, by using the means (10") for detecting a derived time varying magnetic field, a second derived magnetic field resulting from an interaction of the second source magnetic field with the surrounding environment, the means (10") for detecting a derived time varying magnetic field being in a second position and the second derived magnetic field having an amplitude;
- determining, by means of a processor, the position of the passive resonance structure (102) with respect to the means (10") for detecting a derived time varying magnetic field based on the comparison between the amplitude of the first derived magnetic field and the amplitude of the second derived magnetic field, said determining comprising:
   - finding a maximum value between the amplitude of the first derived magnetic field and the amplitude of the second derived magnetic field;
   - identifying a position of interest as the position of the means (10") for detecting a derived magnetic field when detecting an amplitude of the derived magnetic field equal to the maximum value;
   - localizing the nasogastric tube (101) based on the position of interest.

### EXAMPLES

Two types of simulation have been carried out in order to provide a reliable model. Finite element method (FEM) simulation using Ansys Maxwell3D and circuital simulation using Ansys Simplorer. Once the 3D model is simulated with Ansys Maxwell3D, its solution is linked into a circuital model where external conditioning components are added to the model and simulated with Ansys Simplorer.

### Preliminar simulations

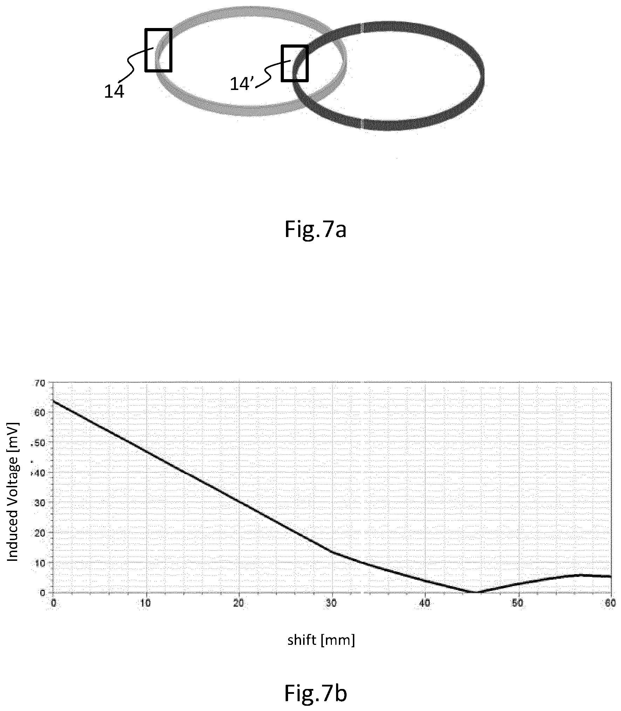

Ansys Maxwell3D allows to simulate the behavior of the coils' electromagnetic fields. In Figure 7a the 3D model is shown which has some intrinsic proprieties like the number of turns that are directly set in the simulation setup.

**Table 1 - Value of the transmitter and Receiver inductors model**

| Inductor (Coil) | Transmitter | Receiver |
|---|---|---|
| Number of turns | 4 | 4 |
| Diameter [mm] | 60 | 60 |
| Material Coil | Copper | Copper |
| Applied Voltage | 10V | 0V |

The model is composed of two coils, as shown in Figure 7a. The gray one is the transmitting coil so an excitation port (14) is applied to it and the black one is the receiving coil and a reading port (14') is applied.

The goal of this simulation is to find the best position for the receiver coil (11') in such a way to obtain a minimum direct coupling between them.

A single point frequency analysis was performed by varying the horizontal position of the receiver coil (11') with respect to the transmitter coil (11). Figure 7b shows the voltage induction vs the position of the receiver coil (11'). In position 0 (shift = 0.00mm, the two coils are completely overlapping) the voltage induction is the maximum since the coils are perfectly coupled. By shifting the receiver coil (11') away, its voltage induction decreases and reaches a minimum in the position of 45.38mm and then increases again until the two coil are completely detached. Our application's goal is to obtain minimum direct coupling between receiver (11') and transmitter (11) coils in absence of any external object (nasogastric tube's resonator) and this happens at an interaxle spacing of 45.38 mm between the two coils.

### Small tag simulations

For the purpose of the present description with the expression "small tag" we intend the tag that has a dimension which is suitable for the nasogastric tube. The "small tag" simulation gives us an understanding of the influence of a real sized sensor on the whole system performance.

The "small tag" simulations are based on the setup of the "preliminary simulations" and the transmitter and receiver coil positions have been kept fixed at the reciprocal distance of 45.38 mm.

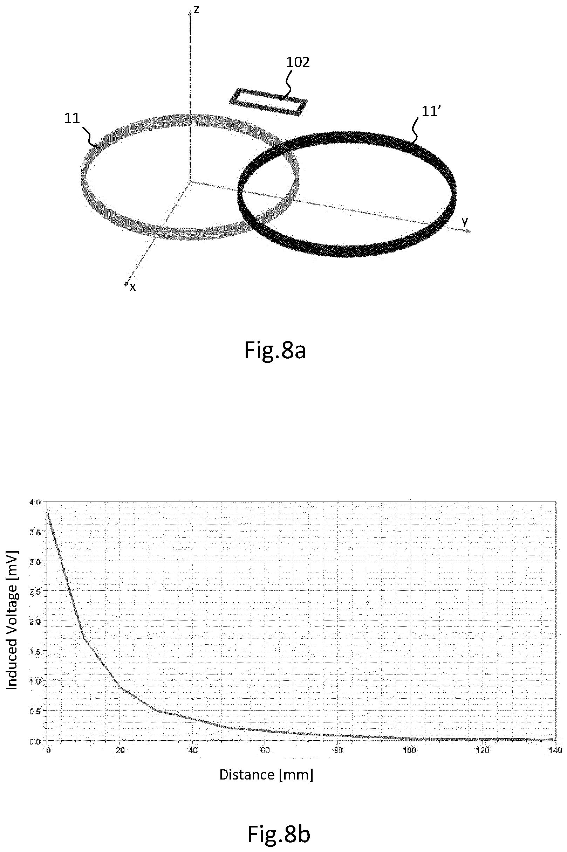

Figure 8a shows the design explained in the "preliminary simulations" paragraph, with the addition of the tag (102).

Parametric simulations are carried out by varying the vertical distance between tag and reading configuration. Without any circuital conditioning (none of the coils is resonating) the voltage inducted is displayed in Figure 8b. As shown in Figure 8b the inducted voltage exponentially decays with the distance from the reader.

### Ansys Simplorer Circuital simulation

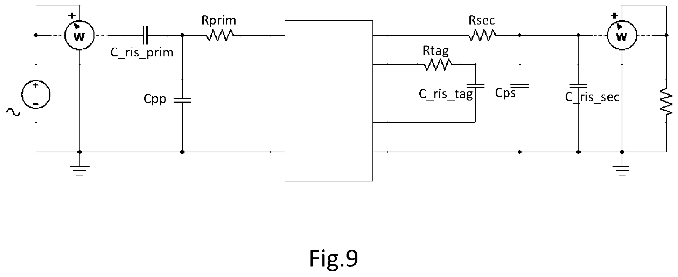

Capacitors are added also here to each coil (see Figure 9), in order make them resonant at 1 MHz.

Two simulations with different configurations are carried out:
1. transmitter coil resonating
2. both transmitter and receiving coils resonating
   (tag coil is resonating in both simulations).

**Table 2 - Setup parameters**

| Parameters | Values |
|---|---|
| Amplitude, frequency | 10V, 1MHz |
| Cris_prim | 11.8nF |
| Cpp | 3.6pF |
| Rprim | 0.4Ω |
| Rsec | 0.4Ω |
| Cps | 3.6pF |
| Cris_sec | 11nF |
| Rtag | 0.3Ω |
| Cris_tag | 32.5nF |

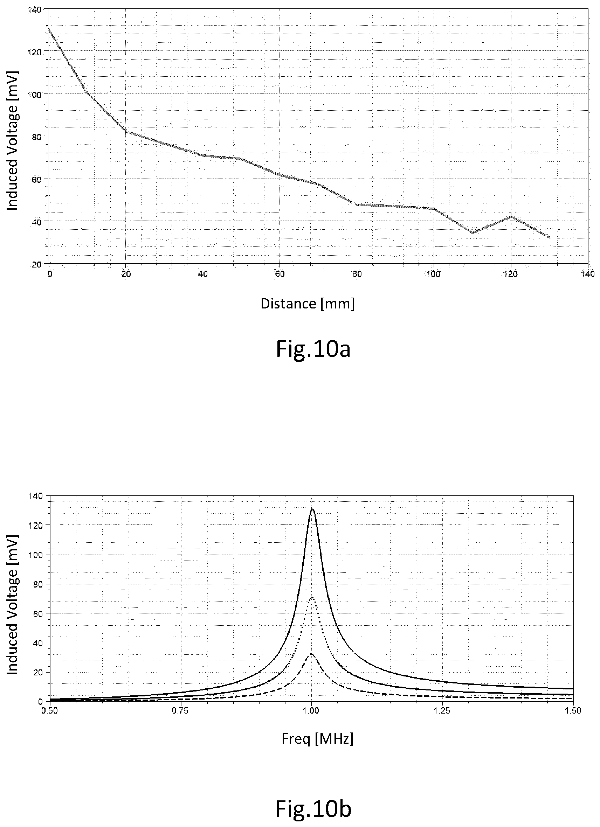

Figure 10a and Figure 10b show the result obtained with the simulation with transmitter coil resonating.

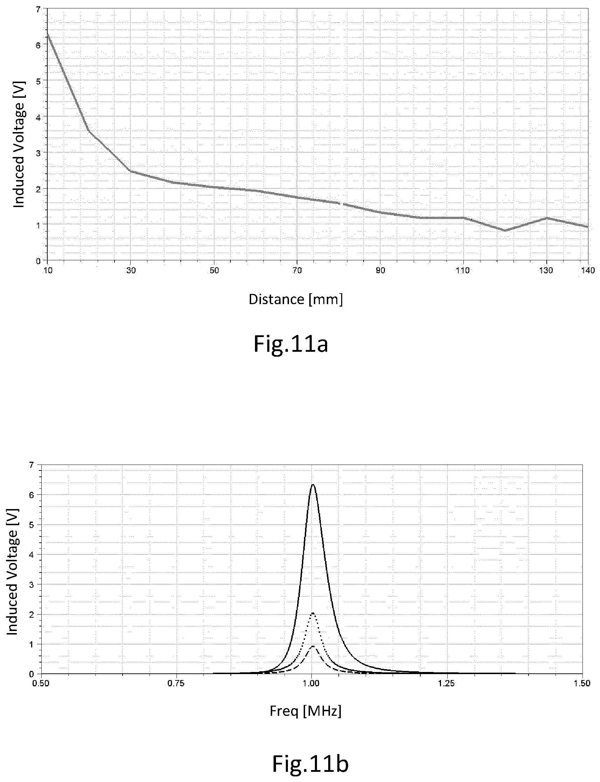

In order to enhance the induced voltage, also the receiving coil is made resonant in the in the Figures 11a and 11b. It is possible to observe that with both transmitter and receiver coils resonating, the signal strength becomes much better (it can be noted that the induced voltage in Figure 10a and 10b is given in milli-volts, whereas that of Figure 11a and Figure 11b is given in volts, i.e. a 1000x factor large).

### Iron tag simulations

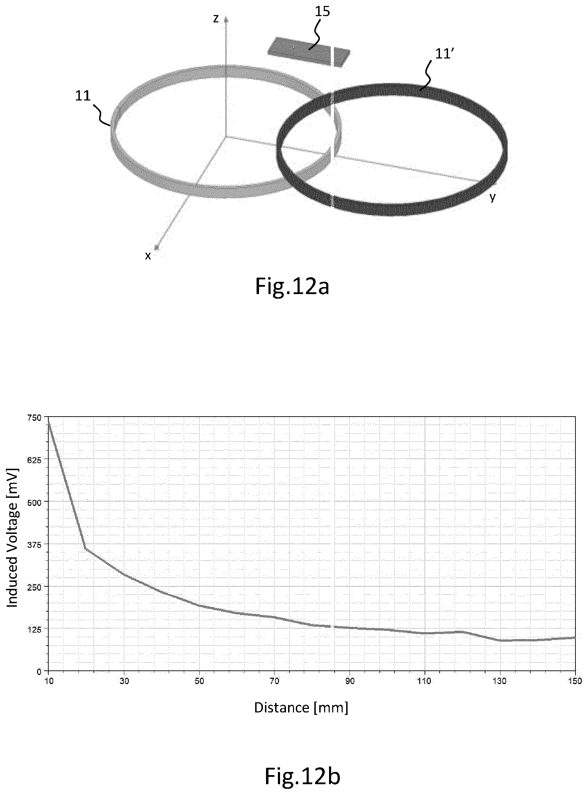

Referring to Figure 12a, the magnetic field produced by the transmitter coil (11) is obviously affected by any nearby conductive object. In order to verify the possibility to distinguish the nasogastric tube from any other metallic object, simulations are carried by considering an iron sheet (15). In this wat, it is possible to comprehend how the receiver coil (11') induced voltage is affected by the iron sheet (15). The iron sheet (15) dimensions are 20x7x1mm.

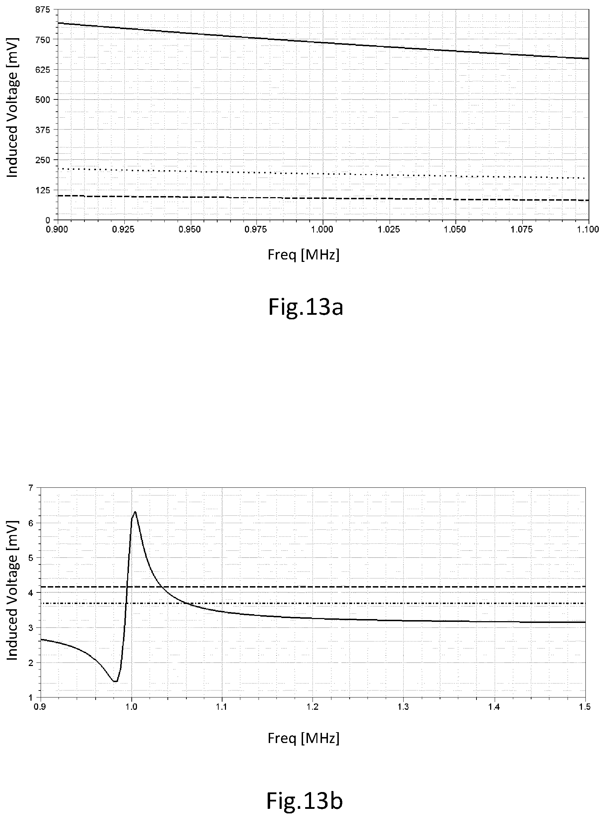

At a single frequency the behavior of the voltage induction is pretty the same as in the case of the small tag. Behavior over various frequencies is however different. The small tag exhibits a marked response at its resonant frequency. Non-resonant objects, on the other hand, have a rather flat response over a wide spectrum of frequencies, as clearly visible in the Figure 13a.

The frequency response in Figure 13a shows that the curves are flat and this means that the iron sheet, and conductive non resonant objects in general, produce a flat frequency response over the investigated frequency band.

In order to distinguish between "iron sheet", "resonant tags" and "no objects at all", at least two frequency points need to be measured.

A comparative simulation is performed to better understand the different frequency responses of a resonant tag, an iron sheet and no object.

Figure 13b displays the frequency response of three real cases. As we can see the frequency response of the iron sheet (dotted line) is flat: this means that it is easy to distinguish. The resonant tag is detectable by its frequency of resonance. Absence of any object is detectable by the very low signal level. Therefore, conducting a two-frequency point analysis will allow to distinguish the resonant tags from any other conductive objects.

### Reader device locating accuracy

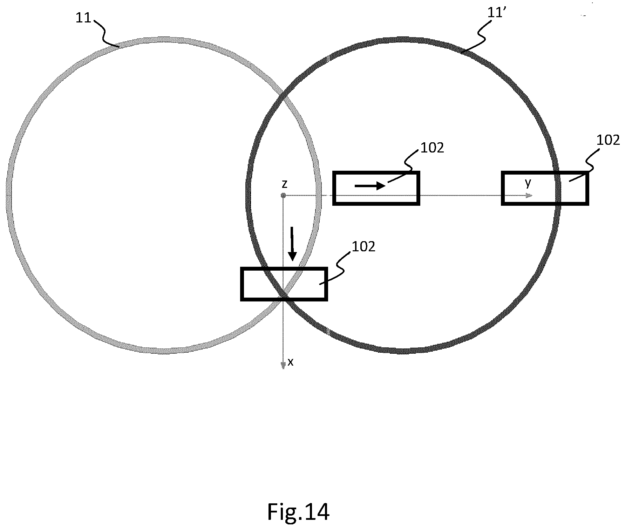

Referring to the Figure 14, to evaluate how well the reader can identify the tag's position, a simulation has been performed where the tag (102) is moved in x and y directions with respect to the reader coils (11, 11').

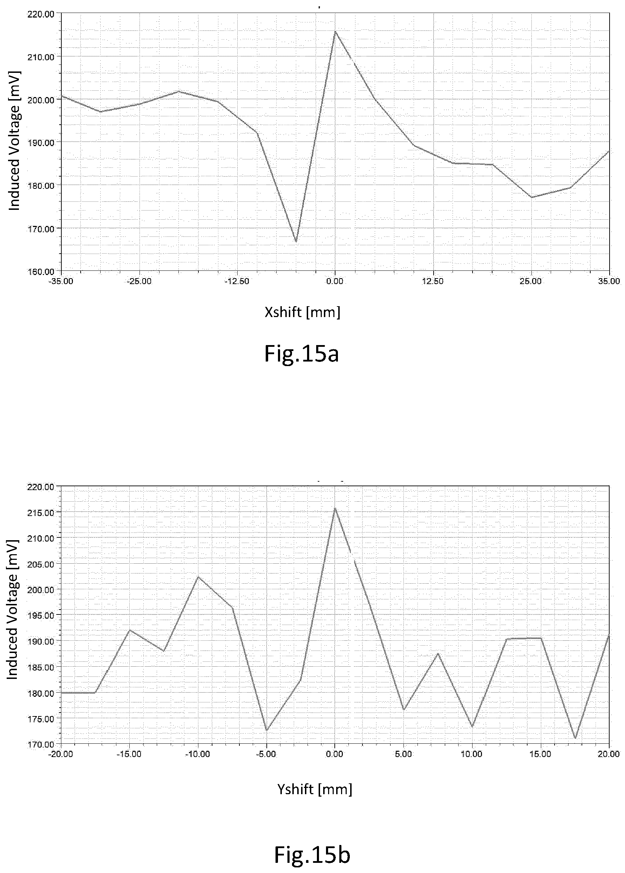

The simulation shows that a change of the tag's position results in a change of the voltage induced in the receiving coil (11'). Adequately processing this information yields an estimated locating accuracy of about ±1cm. As showed in Figure 15a and 15b, showing the effect of the tag sweep on the receiver coil induced voltage, a maximum voltage induction on receiver is obtained when the tag is exactly in the origin position (i.e. right above the reader transmitter and receiver coils).

## Claims

1. A system (10, 100, 200, 300) for enteral feeding (100, 200, 300) comprising:
- a nasogastric tube (101);
- a tag (102) placed on the flexible tube (101), the tag (102) being a passive resonant structure having a resonance frequency band; and
- a reader device (10);
**characterized in that** the reader device (10) comprises:
• means (10') for generating a source time varying magnetic field; and
• means (10") for detecting a derived time varying magnetic field;
and **in that** the tag (102) is configured to interfere with the source magnetic field, by changing its amplitude and/or spatial distribution in such a way to originate the derived magnetic field having its own amplitude and distribution, the source magnetic field having a frequency comprised in the resonance frequency band.

2. The system according to claim 1 wherein the means (10') for generating a source time varying magnetic field comprise at least one first inductor (11) producing the source time varying magnetic field, and the means (10") for detecting the derived magnetic field comprise at least one second inductor (11'), the source time varying magnetic field producing and induced voltage on the at least one second inductor (11'); and wherein the second inductor (11') partially overlapping the at least one first inductor (11) in such a way to cancel out the induced voltage in absence of the tag (102).

3. The system according to any of the preceding claims wherein the tag (102) comprises at least one inductor (103, 203, 303) and at least one capacitor (104, 204, 304).

4. The system according to any of the preceding claims, wherein the means (10') for generating the source magnetic field comprise a capacitor (12)

5. The system according to any of the preceding claims, wherein the means (10") for detecting the derived magnetic field comprise a capacitor (12').

6. The system according to claim 1, wherein the means (10") for detecting a derived time varying magnetic field comprise:
- a bidimensional array of inductors placed at different distance from the tag (102), each of the inductor of the array being placed at its own distance from the tag (102) and producing an output signal dependent on the amplitude and on the distribution of the derived time varying magnetic field; and
- a processor configured for:
• calculating a maximum value of the output signals;
• identifying an inductor of interest as the inductor of the array producing the maximum value; and
• localizing the tag (102) based on the distance of the inductor of interest from the tag (102).

7. The system according to any of the preceding claims wherein the flexible tube (101) has a distal end and proximal end, the tag (102) being placed on the distal end.

8. The system according to any of the preceding claims wherein the resonance frequency band has a central frequency comprised between 100 kHz and 5 MHz.

9. The system according to any of the preceding claims wherein the central frequency is equal to 1 MHz.

10. The system according to any of the claims from 3 to 9 wherein the at least one capacitor (104, 204, 304) is a surface mount technology capacitor.

11. The system according to any of the claims from 3 to 10 wherein the inductor (103, 203, 303) is a coil (103, 203, 303) wrapped at least partially around the tube (101).

12. A method for localizing a nasogastric tube using the system of claim 1, comprising:
- generating a source time varying magnetic field external to the nasogastric tube (101) having its own amplitude and spatial distribution; and
- detecting, by a reader device,a derived magnetic field resulting from an interaction of the first source magnetic field with a surrounding environment;
**characterized in that** the interaction of the source magnetic field with the surrounding environment comprises:
- inducing the resonance of a passive resonance structure (102) placed on the tube (101) and having a resonance frequency band, by means of the source magnetic field, said source magnetic field having a frequency comprised in the resonance frequency band and being generated by the reader device; and
- changing, the amplitude and/or the distribution of the source magnetic field, by means of the passive resonance structure (102), originating the derived magnetic field.

13. The method according to claim 12 comprising:
- generating a first source time varying magnetic field external to the nasogastric tube (101), the first source magnetic field having a first frequency (13) comprised in the resonance frequency band (102);
- detecting a first derived magnetic field resulting from an interaction of the first source magnetic field with the surrounding environment, the first derived magnetic field having an amplitude;
- generating a second source time varying magnetic field external to the nasogastric tube (101), the second source magnetic field having a second frequency (13') outside from the resonance frequency band (102);
- detecting a second derived magnetic field resulting from an interaction of the first source magnetic field with the surrounding environment, the second derived magnetic field having an amplitude;
- determining, by means of a processor, the presence of the passive resonance structure (102), and, thus, of the nasogastric tube (101), based on the difference between the amplitude of the first derived magnetic field and the amplitude of the second derived magnetic field, said determining comprising:
• calculating a difference between the amplitude of the first derived magnetic field and the amplitude of the second derived magnetic field;
• comparing the difference with a fixed threshold;
• identifying the passive resonance structure as present, if the difference is above the fixed threshold; and
• identifying the passive resonance structure as absent, if the difference is below the fixed threshold.

14. The method according to claim 12 comprising:
- generating a first source time varying magnetic field external to the nasogastric tube (101), the first source magnetic field having a frequency comprised in the resonance frequency band;
- detecting, by using means (10") for detecting a derived time varying magnetic field, a first derived magnetic field resulting from an interaction of the first source magnetic field with the surrounding environment, the means (10") for detecting a derived time varying magnetic field being in a first position and the first derived magnetic field having an amplitude;
- moving the means (10") for detecting a derived time varying magnetic field, from a first position to a second position;
- generating a second source time varying magnetic field external to the nasogastric tube (101), the second source magnetic field having a frequency comprised in the resonance frequency band;
- detecting, by using the means (10") for detecting a derived time varying magnetic field, a second derived magnetic field resulting from an interaction of the second source magnetic field with the surrounding environment, the means (10") for detecting a derived time varying magnetic field being in a second position and the second derived magnetic field having an amplitude;
- determining, by means of a processor, the position of the passive resonance structure (102) with respect to the means (10") for detecting a derived time varying magnetic field based on the comparison between the amplitude of the first derived magnetic field and the amplitude of the second derived magnetic field, said determining comprising:
• finding a maximum value between the amplitude of the first derived magnetic field and the amplitude of the second derived magnetic field;
• identifying a position of interest as the position of the means (10") for detecting a derived magnetic field when detecting an amplitude of the derived magnetic field equal to the maximum value;
• localizing the nasogastric tube (101) based on the position of interest.

## Patentansprüche

1. Ein System (10, 100, 200, 300) zur enteralen Ernährung (100, 200, 300), umfassend:
- eine nasogastrale Sonde (101);
- ein Tag (102), das auf der flexiblen Sonde (101) angebracht ist, wobei das Tag (102) eine passive Resonanzstruktur mit einem Resonanzfrequenzband ist; und
- eine Lesevorrichtung;
**dadurch gekennzeichnet, dass** die Lesevorrichtung (10) Folgendes umfasst:
• Mittel (10') zum Erzeugen eines zeitlich variierenden Quellmagnetfelds; und
• Mittel (10") zum Erfassen eines abgeleiteten zeitlich variierenden Magnetfels;
und dadurch, dass das Tag (102) konfiguriert ist, um mit dem Quellmagnetfeld zu interferieren, indem es dessen Amplitude und/oder räumliche Verteilung so verändert, dass ein abgeleitetes Magnetfeld mit eigener Amplitude und Verteilung entsteht, wobei das Quellmagnetfeld eine Frequenz aufweist, die im Resonanzfrequenzband liegt.

2. Das System gemäß Anspruch 1, wobei die Mittel (10') zum Erzeugen eines zeitlich variierenden Magnetfelds mindestens einen ersten Induktor (11) umfassen, der das zeitlich variierende Magnetfeld erzeugt, und die Mittel (10") zum Erfassen des abgeleiteten Magnetfelds mindestens einen zweiten Induktor (11') umfassen, wobei das zeitlich variierende Magnetfeld eine induzierte Spannung an dem mindestens einen zweiten Induktor (11') erzeugt; und wobei der zweite Induktor (11') den mindestens einen ersten Induktor (11) teilweise so überlappt, dass die induzierte Spannung in Abwesenheit des Tags (102) aufgehoben wird.

3. Das System gemäß einem jeden der vorhergehenden Ansprüche, wobei das Tag (102) mindestens einen Induktor (103, 203, 303) und mindestens einen Kondensator (104, 204, 304) umfasst.

4. Das System gemäß einem jeden der vorhergehenden Ansprüche, wobei die Mittel (10') zum Erzeugen des Quellmagnetfelds einen Kondensator (12) umfassen.

5. Das System gemäß einem jeden der vorhergehenden Ansprüche, wobei die Mittel (10") zum Erfassen des abgeleiteten Magnetfelds einen Kondensator (12') umfassen.

6. Das System gemäß Anspruch 1, wobei die Mittel (10") zum Erfassen eines abgeleiteten zeitlich variierenden Magnetfelds Folgendes umfassen:
- ein zweidimensionales Array von Induktoren, die in unterschiedlichem Abstand vom Tag (102) angeordnet sind, wobei jeder Induktor des Arrays in einem eigenen Abstand vom Tag (102) angeordnet ist und ein Ausgangssignal erzeugt, das von der Amplitude und der Verteilung des abgeleiteten zeitlich variierenden Magnetfelds abhängt; und
- einen Prozessor, der für Folgendes konfiguriert ist:
• Berechnen eines Maximalwerts der Ausgangssignale;
• Identifizieren eines Induktors von Interesse als den Induktor des Arrays, der den Maximalwert erzeugt; und
• Lokalisieren des Tags (102) auf der Basis des Abstands des Induktors von Interesse vom Tag (102).

7. Das System gemäß einem jeden der vorhergehenden Ansprüche, wobei die flexible Sonde (101) ein distales Ende und ein proximales Ende aufweist, wobei das Tag (102) am distalen Ende angeordnet ist.

8. Das System gemäß einem jeden der vorhergehenden Ansprüche, wobei das Resonanzfrequenzband eine zentrale Frequenz zwischen 100 kHz und 5 MHz aufweist.

9. Das System gemäß einem jeden der vorhergehenden Ansprüche, wobei die zentrale Frequenz gleich 1 MHz ist.

10. Das System gemäß einem jeden der Ansprüche von 3 bis 9, wobei der mindestens eine Kondensator (104, 204, 304) ein Kondensator in Oberflächenmontage ist.

11. Das System gemäß einem jeden der Ansprüche von 3 bis 10, wobei der Induktor (103, 203, 303) eine Spule (103, 203, 303) ist, die zumindest teilweise um die Sonde (101) gewickelt ist.

12. Eine Methode zur Lokalisierung einer nasogastralen Sonde unter Verwendung des Systems gemäß Anspruch 1, umfassend:
- Erzeugen eines zeitlich variierenden Magnetfelds außerhalb der nasogastralen Sonde (101), die eine eigene Amplitude und räumliche Verteilung besitzt; und
- Erfassen, durch ein Lesevorrichtung, eines abgeleiteten Magnetfelds, das aus einer Wechselwirkung des ersten Quellmagnetfelds mit der Umgebung resultiert;
**dadurch gekennzeichnet, dass** die Wechselwirkung des Quellmagnetfelds mit der Umgebung Folgendes umfasst:
- Induzieren der Resonanz einer passiven Resonanzstruktur (102), die auf der Sonde (101) angeordnet ist und ein Resonanzfrequenzband aufweist, mittels des Quellmagnetfelds, wobei das besagte Quellmagnetfeld eine Frequenz aufweist, die in dem Resonanzfrequenzband enthalten ist, und von der Lesevorrichtung erzeugt wird; und
- Ändern der Amplitude und/oder der Verteilung des Quellmagnetfelds mittels der passiven Resonanzstruktur (102), die das abgeleitete Magnetfeld erzeugt.

13. Die Methode gemäß Anspruch 12, umfassend:
- Erzeugen eines ersten zeitlich variierenden Magnetfelds außerhalb der nasogastralen Sonde (101), wobei das erste Quellmagnetfeld eine erste Frequenz (13) aufweist, die im Resonanzfrequenzband (102) liegt;
- Erfassen eines ersten abgeleiteten Magnetfelds, das aus einer Wechselwirkung des ersten Quellenmagnetfelds mit der Umgebung resultiert, wobei das erste abgeleitete Magnetfeld eine Amplitude aufweist;
- Erzeugen eines zweiten zeitlich variierenden Magnetfelds außerhalb der nasogastralen Sonde (101), wobei das zweite Quellmagnetfeld eine zweite Frequenz (13') außerhalb des Resonanzfrequenzbandes (102) aufweist;
- Erfassen eines zweiten abgeleiteten Magnetfelds, das aus einer Wechselwirkung des ersten Quellmagnetfelds mit der Umgebung resultiert, wobei das zweite abgeleitete Magnetfeld eine Amplitude aufweist;
- Bestimmen, mittels eines Prozessors, des Vorhandenseins der passiven Resonanzstruktur (102) und somit der nasogastralen Sonde (101) auf der Basis der Differenz zwischen der Amplitude des ersten abgeleiteten Magnetfelds und der Amplitude des zweiten abgeleiteten Magnetfelds, wobei das besagte Bestimmen Folgendes umfasst:
• Berechnen einer Differenz zwischen der Amplitude des ersten abgeleiteten Magnetfelds und der Amplitude des zweiten abgeleiteten Magnetfelds;
• Vergleichen der Differenz mit einem festen Schwellenwert;
• Identifizieren der passiven Resonanzstruktur als vorhanden, wenn die Differenz über dem festen Schwellenwert liegt; und
• Identifizieren der passiven Resonanzstruktur als nicht vorhanden, wenn die Differenz unter dem festen Schwellenwert liegt.

14. Die Methode gemäß Anspruch 12, umfassend:
- Erzeugen eines ersten zeitlich variierenden Magnetfelds außerhalb der nasogastralen Sonde (101), wobei das erste Quellmagnetfeld eine Frequenz aufweist, die im Resonanzfrequenzband liegt;
- Erfassen, unter Verwendung von Mitteln (10") zum Erfassen eines abgeleiteten zeitlich variierenden Magnetfelds, eines ersten abgeleiteten Magnetfelds, das aus einer Wechselwirkung des ersten Quellmagnetfelds mit der Umgebung resultiert, wobei sich die Mittel (10") zum Erfassen eines abgeleiteten zeitlich variierenden Magnetfelds in einer ersten Position befinden und das erste abgeleitete Magnetfeld eine Amplitude aufweist;
- Bewegen der Mittel (10") zum Erfassen eines abgeleiteten zeitlich variierenden Magnetfelds von einer ersten Position zu einer zweiten Position;
- Erzeugen eines zweiten zeitlich variierenden Quellmagnetfelds außerhalb der Magensonde (101), wobei das zweite Magnetfeld eine Frequenz aufweist, die im Resonanzfrequenzband liegt;
- Erfassen, unter Verwendung von Mitteln (10") zum Erfassen eines abgeleiteten zeitlich variierenden Magnetfelds, eines zweiten abgeleiteten Magnetfelds, das aus einer Wechselwirkung des zweiten Quellmagnetfelds mit der Umgebung resultiert, wobei sich die Mittel (10") zum Erfassen eines abgeleiteten zeitlich variierenden Magnetfelds in einer zweiten Position befinden und das zweite abgeleitete Magnetfeld eine Amplitude aufweist;
- Bestimmen, mittels eines Prozessors, der Position der passiven Resonanzstruktur (102) in Bezug auf die Mittel (10") zum Erfassen eines abgeleiteten zeitlich variierenden Magnetfelds auf der Basis des Vergleichs zwischen der Amplitude des ersten abgeleiteten Magnetfelds und der Amplitude des zweiten abgeleiteten Magnetfelds, wobei das besagte Bestimmen Folgendes umfasst:
• Bestimmen eines Maximalwerts zwischen der Amplitude des ersten abgeleiteten Magnetfelds und der Amplitude des zweiten abgeleiteten Magnetfelds;
• Identifizieren einer Position von Interesse als die Position der Mittel (10") zum Erfassen eines abgeleiteten Magnetfelds, wenn eine Amplitude des abgeleiteten Magnetfels erfasst wird, die dem Maximalwert entspricht;
• Lokalisieren der nasogastralen Sonde (101) auf der Basis der Position von Interesse.

## Revendications

1. Système (10, 100, 200, 300) pour l'alimentation entérale (100, 200, 300) comprenant :
- une sonde nasogastrique (101) ;
- une étiquette (102) placée sur la sonde flexible (101), l'étiquette (102) étant une structure résonante passive ayant une bande de fréquences de résonance ; et
- un dispositif de lecture (10) ;
**caractérisé par le fait que** le dispositif de lecture (10) comprend :
• des moyens (10') de génération d'un champ magnétique source variant dans le temps ; et
• des moyens (10") de détection d'un champ magnétique dérivé variant dans le temps ;
et **par le fait que** l'étiquette (102) est configurée pour interférer avec le champ magnétique source, en modifiant son amplitude et/ou sa distribution spatiale de manière à créer le champ magnétique dérivé ayant sa propre amplitude et sa propre distribution, le champ magnétique source ayant une fréquence comprise dans la bande de fréquences de résonance.

2. Système selon la revendication 1, où les moyens (10') de génération d'un champ magnétique variable dans le temps comprennent au moins une première inductance (11) produisant le champ magnétique variable dans le temps, et les moyens (10") de détection du champ magnétique dérivé comprennent au moins une deuxième inductance (11'), le champ magnétique variable dans le temps produisant une tension induite sur la au moins une deuxième inductance (11') ; et où le deuxième inducteur (11') chevauche partiellement le au moins un premier inducteur (11) de manière à annuler la tension induite en l'absence de l'étiquette (102).

3. Système selon l'une des revendications précédentes, où l'étiquette (102) comprend au moins une inductance (103, 203, 303) et au moins un condensateur (104, 204, 304).

4. Système selon l'une des revendications précédentes, où le moyen (10') de génération du champ magnétique source comprend un condensateur (12).

5. Système selon l'une des revendications précédentes, où le moyen (10") de détection du champ magnétique dérivé comprend un condensateur (12').

6. Système selon la revendication 1, où les moyens (10") de détection d'un champ magnétique dérivé variant dans le temps comprennent :
- un réseau bidimensionnel d'inductances placées à différentes distances de l'étiquette (102), chacune des inductances du réseau étant placée à sa propre distance de l'étiquette (102) et produisant un signal de sortie dépendant de l'amplitude et de la distribution du champ magnétique dérivé variant dans le temps ; et
- un processeur configuré pour :
• calculer une valeur maximale des signaux de sortie ;
• identifier une inductance d'intérêt comme étant l'inductance du réseau produisant la valeur maximale ; et
• localiser l'étiquette (102) sur la base de la distance entre l'inductance d'intérêt et l'étiquette (102).

7. Système selon l'une des revendications précédentes, où le tube flexible (101) comporte une extrémité distale et une extrémité proximale, l'étiquette (102) étant placée sur l'extrémité distale..

8. Système selon l'une des revendications précédentes, où la bande de fréquences de résonance a une fréquence centrale comprise entre 100 kHz et 5 MHz.

9. Système selon l'une des revendications précédentes, où la fréquence centrale est égale à 1 MHz.

10. Système selon l'une des revendications 3 à 9, où le ou les condensateurs (104, 204, 304) sont des condensateurs à montage en surface.

11. Système selon l'une des revendications 3 à 10, où l'inductance (103, 203, 303) est une bobine (103, 203, 303) enroulée au moins partiellement autour du tube (101).

12. Procédé de localisation d'une sonde nasogastrique à l'aide du système selon la revendication 1, comprenant :
- la génération d'un champ magnétique source variant dans le temps à l'extérieur de la sonde nasogastrique (101) ayant sa propre amplitude et sa propre distribution spatiale ; et
- la détection, par un dispositif de lecture, d'un champ magnétique dérivé résultant d'une interaction du premier champ magnétique source avec un environnement environnant ;
**caractérisé par le fait que** l'interaction du champ magnétique source avec l'environnement environnant comprend :
- l'induction de la résonance d'une structure de résonance passive (102) placée sur la sonde (101) et ayant une bande de fréquences de résonance, au moyen du champ magnétique source, ledit champ magnétique source ayant une fréquence comprise dans la bande de fréquences de résonance et étant généré par le dispositif de lecture ; et
- la modification de l'amplitude et/ou de la distribution du champ magnétique source, au moyen de la structure résonante passive (102), à l'origine du champ magnétique dérivé.

13. Procédé selon la revendication 12 comprenant :
- la génération d'un premier champ magnétique variable dans le temps externe à la sonde nasogastrique (101), le premier champ magnétique ayant une première fréquence (13) comprise dans la bande de fréquences de résonance (102) ;
- la détection d'un premier champ magnétique dérivé résultant d'une interaction du premier champ magnétique source avec l'environnement environnant, le premier champ magnétique dérivé ayant une amplitude ;
- la génération d'un deuxième champ magnétique source variant dans le temps à l'extérieur de la sonde nasogastrique (101), le deuxième champ magnétique source ayant une deuxième fréquence (13') en dehors de la bande de fréquences de résonance (102) ;
- la détection d'un deuxième champ magnétique dérivé résultant d'une interaction du premier champ magnétique source avec l'environnement environnant, le deuxième champ magnétique dérivé ayant une amplitude ;
- la détermination, au moyen d'un processeur, de la présence de la structure de résonance passive (102), et donc de la sonde nasogastrique (101), sur la base de la différence entre l'amplitude du premier champ magnétique dérivé et l'amplitude du deuxième champ magnétique dérivé, ladite détermination comprenant :
• le calcul d'une différence entre l'amplitude du premier champ magnétique dérivé et l'amplitude du deuxième champ magnétique dérivé ;
• la comparaison de la différence avec un seuil fixe ;
• l'identification de la structure de résonance passive comme présente, si la différence est supérieure au seuil fixe ; et
• l'identification de la structure de résonance passive comme absente, si la différence est inférieure au seuil fixe.

14. Procédé selon la revendication 12 comprenant :
- la génération d'un premier champ magnétique variable dans le temps externe à la sonde nasogastrique (101), le premier champ magnétique ayant une fréquence comprise dans la bande de fréquences de résonance ;
- la détection, à l'aide de moyens (10") de détection d'un champ magnétique dérivé variant dans le temps, d'un premier champ magnétique dérivé résultant d'une interaction du premier champ magnétique source avec l'environnement environnant, les moyens (10") de détection d'un champ magnétique dérivé variant dans le temps étant dans une première position et le premier champ magnétique dérivé ayant une amplitude ;
- le déplacement des moyens (10") de détection d'un champ magnétique dérivé variant dans le temps, d'une première position à une deuxième position;
- la génération d'un deuxième champ magnétique source variant dans le temps à l'extérieur de la sonde nasogastrique (101), le deuxième champ magnétique source ayant une fréquence comprise dans la bande de fréquences de résonance ;
- la détection, à l'aide des moyens (10") de détection d'un champ magnétique dérivé variant dans le temps, d'un deuxième champ magnétique dérivé résultant d'une interaction du deuxième champ magnétique source avec l'environnement environnant, les moyens (10") de détection d'un champ magnétique dérivé variant dans le temps se trouvant dans une deuxième position et le deuxième champ magnétique dérivé ayant une amplitude ;
- la détermination, au moyen d'un processeur, de la position de la structure de résonance passive (102) par rapport aux moyens (10") de détection d'un champ magnétique dérivé variant dans le temps, sur la base de la comparaison entre l'amplitude du premier champ magnétique dérivé et l'amplitude du deuxième champ magnétique dérivé, ladite détermination comprenant :
• la recherche d'une valeur maximale entre l'amplitude du premier champ magnétique dérivé et l'amplitude du deuxième champ magnétique dérivé ;
• l'identification d'une position d'intérêt comme étant la position des moyens (10") de détection d'un champ magnétique dérivé lors de la détection d'une amplitude du champ magnétique dérivé égale à la valeur maximale ;
• la localisation de la sonde nasogastrique (101) sur la base de la position d'intérêt.
